# EUROPÄISCHE PATENTSCHRIFT

(11) **EP 1 235 775 B1**
(45) Veröffentlichungstag und Bekanntmachung des Hinweises auf die Patenterteilung: **19.05.2004**
(21) Anmeldenummer: 00987276.3
(22) Anmeldetag: 21.11.2000
(51) Int. Cl.: C07C 29/56, C07C 33/02

(54) **VERFAHREN ZUR ISOMERISIERUNG VON ALLYLALKOHOLEN**
METHOD FOR ISOMERISING ALLYL ALCOHOLS
PROCEDE POUR L'ISOMERISATION D'ALCOOLS ALLYLIQUES

(30) Priorität: 06.12.1999 DE 19958603
(43) Veröffentlichungstag der Anmeldung: 04.09.2002
(73) Patentinhaber: BASF AKTIENGESELLSCHAFT, 67056 Ludwigshafen (DE)
(72) Erfinder: EBEL, Klaus, 68623 Lampertheim (DE); STOCK, Frank, 67227 Frankenthal (DE)
(86) Internationale Anmeldenummer: PCT/EP2000/011580
(87) Internationale Veröffentlichungsnummer: WO 2001/042177

(56) Entgegenhaltungen:
- DE-A- 2 516 698
- GB-A- 1 256 184
- P. CHARBARDES, ET AL.: "Use of oxo-metallic derivatives in isomerisation" TETRAHEDRON LETTERS, Bd. 33, Nr. 14, 1977, Seiten 1775-1783, XP002165950 Elsevier Science Publishers, Amsterdam, NL ISSN: 0040-4039 in der Anmeldung erwähnt
- T. HOSOGAI, ET AL.: "Selective allyic rearrangement with tungsten catalyst" CHEMISTRY LETTERS, Nr. 3, März 1982 (1982-03), Seiten 357-360, XP002165951 Chemical Society of Japan, Tokyo, JP ISSN: 0366-7022 in der Anmeldung erwähnt

## Beschreibung

Die vorliegende Erfindung betrifft ein verbessertes Verfahren zur Isomerisierung von Edukt-Allylalkoholen zu Produkt-Allylalkoholen in beiden Richtungen des Gleichgewichtes in Gegenwart von Wolframoxo (VI)-Komplexen enthaltend zusätzliche Stickstoff enthaltende Liganden.

Allylalkohole sind wichtige Zwischenprodukte in der industriellen organischen Chemie. Tertiäre Allylalkohole insbesondere dienen zum Beispiel als Zwischenstufen bei der Herstellung von Riechstoffen oder auch als Additive in Seifen oder Detergentien.

Es ist bekannt, daß Allylalkohole unter Säurekatalyse isomerisieren. Diese Isomerisierung entspricht einer 1,3-Wanderung der Hydroxigruppe und einer entsprechenden Verschiebung der Doppelbindung, wie in der folgenden Gleichung mit den allgemeinen Formeln I und II dargestellt: 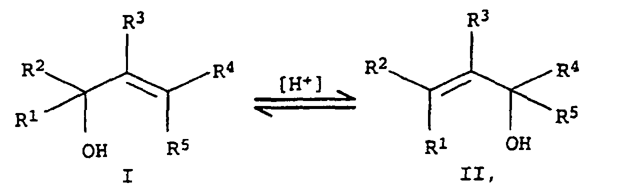 in denen R¹ bis R⁵ für Wasserstoff oder Kohlenwasserstoffreste stehen.

Das Verfahren ist besonders geeignet für die Herstellung von tertiären Produkt-Allylalkoholen, wie 2-Linalool, durch Isomerisieren von primären oder sekundären Allylalkoholen, wie Geraniol und Nerol.

Geraniol (2-trans-3,7-Dimethyl-2,6-octadien-8-ol), Nerol (2-cis-3,7-Dimethyl-2,6-octadien-8-ol) und 2-Linalool (3,7-Dimethyl-1,6-octadien-3-ol) sind wichtige Verbindungen in der Riechstoffindustrie. Sie werden entweder direkt als Riechstoffe verwendet oder durch Umsetzung mit anderen Verbindungen zu höher molekularen Riechstoffen umgesetzt. Bedeutung besitzen diese Terpenalkohole auch als C₁₀-Bausteine bei der Synthese von Vitaminen, wie dem Vitamin E.

In der Literatur wurden in der Vergangenheit bevorzugt Verfahren zur Isomerisierung von Linalool zu Geraniol beschrieben. Da es sich bei den Isomerisierungen um Gleichgewichtsreaktionen handelt, sind die entwickelten Verfahren prinzipiell auch für die umgekehrte Reaktion der Isomerisierung von Geraniol oder Nerol zu Linalool anwendbar.

Zunächst wurden die Isomerisierungsreaktionen von Allylalkoholen mit Säuren als Katalysatoren durchgeführt. Diese Verfahren waren jedoch nur von eingeschränkter Bedeutung da bei ihnen Nebenreaktionen, wie z.B. Dehydratisierungen oder Cyclisierungen überwiegen.

Später wurde die katalytische Umlagerung von substituierten Allylalkoholen mit Molybdän-, Vanadium- und Wolfram-Katalysatoren untersucht (vgl. P. Chabardes et al in *Tetrahedron* 33 (1977), Seiten 1775-1783.).

Während die in GB 1 256 184 als Isomerisierungskatalysator beschriebene Molybdänverbindung unbefriedigende Reaktionsergebnisse ergab, waren mit Wolframoxo (VI) alkoholat-Katalysatoren der Formel WO(OR)₄ in Gegenwart einer Stickstoff-Base als zusätzlichem Liganden relativ hohe Selektivitäten bei gleichzeitig höheren Aktivitäten, verglichen mit den analogen Vanadiumoxo(V)alkoholat-Katalysatoren der Formel VO(OR)₃ möglich. Weitere Vorteile der Wolfram-Katalysatoren liegen darin, daß sie sich leicht aus dem Reaktionsgemisch abtrennen lassen (vgl. T. Hosogai et al in *Chemistry Letters* 1982, Seiten 357-360.) und daß sie im Vergleich zum Vanadiumkatalysator eine nur geringe Toxizität aufweisen.

Weiterhin ist aus DE 25 16 698 die Herstellung von neuen Katalysatoren auf der Basis von Wolfram, sowie deren Verwendung für die katalytische Umlagerung von tertiären zu primären Allylalkoholen bekannt. Als Katalysatoren werden bei diesem Verfahren Wolframoxo(VI)-Komplexe enthaltend Alkoxy-Reste und/oder über Sauerstoff gebundene Trialkylsilyl-Reste beschrieben, die zur Verbesserung der Selektivität zusätzlich koordinativ an das Wolfram gebundene Liganden enthalten, welche ein Element ausgewählt aus den Elementen N, P, As und Bi, insbesondere Liganden ausgewählt aus der Klasse der primären, sekundären und tertiären Monoamine, der Polyamine, der Schiff'sehen Basen, der Imine, Nitrile und Isonitrile enthalten. Als besonders geeignete Liganden werden hierin genannt primäre Monoamine, wie Methylamin, Ethylamin, Propylamin, β-Ethoxy-ethylamin, Butylamin, Cyclohexylamin, Anilin und Naphthylamin; sekundäre Monoamine, wie Dimethylamin, Diethylamin, Dibutylamin, Dicyclohexylamin, Methylanilin, Methylcyclohexylamin, Piperidin, Morpholin und Pyrrolidin; tertiäre Monoamine, wie Trimethylamin, Triethylamin, Ethyldibutylamin, Tricyclohexylamin, Dimethylanilin, Pyridin, Pikolin, Chinolin, Isochinolin, N-Methylpyrrolidin und N-Methyl-morpholin; Ethylendiamin,

Pyrazin, Piperazin, Pyrimidin, Triethylendiamin, 1,8-Diaza-bicyclo[5,4,0]undec-7-en, Polyethylenimine sowie Ionenaustauscherharze mit einer Vielzahl von Aminogruppen innerhalb der Moleküle, insbesondere Pyridin, Triethylamin, Cyclohexylamin, Diethylamin und Tricyclohexylphosphin. Aminoalkohole werden hierin nicht genannt. Die mit diesem Verfahren erzielten Selektivitäten an primären Alkoholen, wie Nerol und Geraniol, sind recht gut, doch lassen die dabei erzielten Umsätze noch zu wünschen übrig.

Eigene Untersuchungen zur Isomerisierung von Geraniol mit einer 0,05 mol-%-igen Lösung von Wolframoxo(VI)-tetrakisgeranylat analog zum dem in DE 25 16 698 beschriebenen Verfahren zeigten, daß die Umlagerung zu Linalool in Gegenwart einer Stickstoff-Base bei 200°C (Reaktionszeit etwa 1 Stunde) deutlich selektiver verläuft als ohne Mitverwendung einer Stickstoff-Base (siehe Vergleichsbeispiele 1 bis 5).

Als Stickstoffbasen wurden hierbei Diethylamin, Pyridin, Imidazol und Poly-(4-vinyl-pyridin) eingesetzt.

Nachteilig wirkten sich bei diesen Versuchen die vergleichsweise geringeren Umsätze bei gleichzeitig hohen Temperaturen von über 150°C aus, welche die Bildung von Nebenprodukten beschleunigten.

Es war daher die Aufgabe der Erfindung das Verfahren zur Isomerisierung von Allylalkoholen in Gegenwart von Wolframoxo(VI)-Komplexen enthaltend zusätzliche Stickstoff enthaltende Liganden bei Temperaturen von 50 bis 300°C so zu verbessern, daß auch bei der Isomerisierung von primären oder sekundären Allylalkoholen, wie Geraniol und Nerol, zu tertiären Allylalkoholen, wie Linalool, höhere Umsätze des eingesetzten Edukt-Allylalkohols erzielt werden, wodurch die Geschwindigkeit der Gleichgewichtseinstellung beschleunigt wird und die Raum-Zeit-Ausbeuten steigen.

Es wurde überraschenderweise gefunden, daß das Verfahren zur Isomerisierung von Edukt-Allylalkoholen zu Produkt-Allylalkoholen in Gegenwart von homogen gelösten Wolframoxo(VI)-Komplexen enthaltend zusätzliche Stickstoff enthaltende Liganden bei gleichbleibenden Selektivitäten höhere Aktivitäten für die Isomerisierung von Geraniol und Nerol zu Linalool ergeben als entsprechende Isomerisierungen mit Molframoxo (VI) alkoholat-Katalysatoren in Gegenwart der bekannten Stickstoff-Basen allein, wenn man als zusätzliche Stickstoff enthaltende Liganden Aminoalkohole verwendet.

Eine höhere Aktivität bei der Allylumlagerung ergibt sich auch dann, wenn die Aminoalkohole erst nachträglich zu Wolframoxo (VI) alkoholat-Katalysatoren der Formel WO(OR)₄ zugegeben werden, denen bereits Stickstoff-Basen als zusätzliche Liganden zugefügt wurden.

Gegenstand der Erfindung ist dementsprechend ein Verfahren zur Isomerisierung von Edukt-Allylalkoholen zu Produkt-Allylalkoholen in Gegenwart von entweder vorgefertigten oder in situ erzeugten wolframoxo (VI)-Komplexen enthaltend zusätzliche Stickstoff enthaltende Liganden bei Temperaturen von 50 bis 300°C, das dadurch gekennzeichnet ist, daß in dem Katalysator neben den als zusätzliche Stickstoff enthaltende Liganden bekannten Stickstoffbasen oder anstelle dieser Stickstoffbasen als zusätzliche Stickstoff enthaltende Liganden Aminoalkohole enthalten sind.

Als mit Hilfe des erfindungsgemäßen Verfahrens vorteilhaft isomerisierbare Allylalkohole seien beispielsweise genannt:
2-Methyl-3-buten-2-ol, Prenol (3-Methyl-2-buten-1-ol), Linalool, Nerol und Geraniol sowie Farnesol (3,7,11-Trimethyl-dodeca-2,6,10-trien-1-ol) und Nerolidol (3,7,11-Trimethyl-dodeca-1,6,10-trien-3-ol), insbesondere Linalool, Nerol und Geraniol.

Als Aminoalkohole können bei dem erfindungsgemäßen Verfahren Aminoalkohole der allgemeinen Formel III verwendet werden. 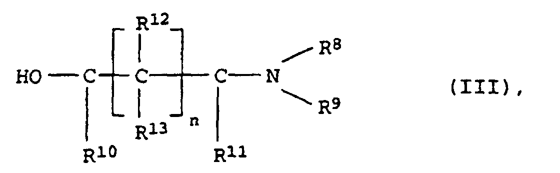
- in der R⁸ und R⁹: gleich oder verschieden sind und Wasserstoff, C₁- bis C₄-Alkyl, -CH₂-C₆H₅OH; -C₂H₅OH oder - C₃H₇OH bedeuten,
- R¹⁰ und R¹¹: gleich oder verschieden sind und H, C₁- bis C₄-Alkyl bedeuten oder aber zusammen für einen gesättigten oder ungesättigten, ggf. mit C₁- bis C₄-Alkylgruppen substituierten zweiwertigen Rest bilden,
- R¹² und R¹³: gleich oder verschieden sind und für Wasserstoff oder einen C₁- bis C₄-Alkylrest stehen,
- und n: für eine ganze Zahl von 0 bis 5 steht.

Mit besonderem Vorteil gelingt das erfindungsgemäße Verfahren, wenn man als Aminoalkohole Triethanolamin, Diethanolamin, Monoethanolamin, Tripropanolamin, Dipropanolamin, 3-Amino-1-propanol, 1-Amino-2-propanol, 2-Amino-1-propanol, Butyldiethanolamin, Methyldiisopropanolamin, N-(2-Hydroxy-benzyl)-amin oder N,N'-Bis-(2-hydroxy-benzyl)-1,2-diaminoethan verwendet.

Zur Herstellung der für das erfindungsgemäße Verfahren benötigten Isomerisierungskatalysatoren verwendet man als Wolframoxo(VI)-Komplexe im allgemeinen die in DE 25 16 698 beschriebenen Wolframoxo(VI)-Komplexe bzw. Wolfram(VI)-Verbindungen der allgemeinen Formeln

[WO (OR¹) (OR²) (OR³) (OR⁴)]ₙ L

oder

WO (OR¹) (OR²) (OR³) (OR⁴),

in denen R¹ bis R⁴ für Kohlenwasserstoffgruppen, substituierte Kohlenwasserstoffgruppen oder Gruppen der Formel 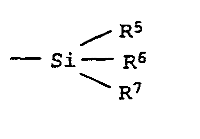 stehen,
wobei R⁵, R⁶ und R⁷ jeweils Kohlenwasserstoffgruppen bedeuten, die gegebenenfalls substituiert sein können, wobei R¹, R², R3 und R⁴ gleich oder verschieden sein können und, falls zwei oder mehrere der Gruppen R¹, R², R³ und R⁴ Kohlenwasserstoffgruppen sind, unabhängig davon, ob sie substituiert oder nicht-substituiert sind, zwei derartige Gruppen eine cyclische Struktur zusammen mit den benachbarten Wolfram- und Sauerstoffatomen in der Formel bilden können, vorzugsweise Verbindungen, in denen R¹, R², R³, R⁴, R⁵, R⁶ und R⁷ Kohlenwasserstoffgruppen, ausgewählt aus folgenden Gruppen bedeuten: Alkylgruppen mit 1 bis 20 Kohlenstoffatomen, Alkenylgruppen mit 2 bis 20 Kohlenstoffatomen, Cycloalkylgruppen mit 3 bis 10 Kohlenstoffatomen, Cycloalkenylgruppen mit 3 bis 10 Kohlenstoffatomen, Arylgruppen mit 6 bis 10 Kohlenstoffatomen, Aralkylgruppen mit 7 bis 20 Kohlenstoffatomen sowie Alkylarylgruppen mit 7 bis 20 Kohlenstoffatomen, insbesondere Verbindungen, in denen R¹ und R² die oben angegebene Bedeutung haben, R3 und R⁴ für Wasserstoff oder eine Methylgruppe stehen oder zusammen einen gesättigten oder ungesättigten zweiwertigen Rest bilden und R⁵, R⁶ und R⁷ Wasserstoff bedeuten, L ein einwertiger oder mehrwertiger Ligand ist, der Stickstoff enthält und über diesen Stickstoff mit dem Wolframatom in der Formel koordinativ verbunden ist, und n ein Wert ist, der zwischen 1 und der Anzahl der mit dem Liganden L koordinativ verbindbaren Wolframatome liegt.
Die Wolframoxo(VI)-Komplexe können beispielsweise gemäß den in DE 25 16 698 beschriebenen Verfahren aus Wolframtrioxid mit Hydroxyverbindungen, die Gruppen enthalten, welche R¹, R², R³ und R⁴ entsprechen, in Gegenwart der Ligand-Verbindung; oder durch Umsetzen eines Wolframoxytetrahalogenids mit einem Alkalimetallalkoholat einer Hydroxyverbindung mit Gruppen, die R¹, R², R³ und R⁴ entsprechen, in Gegenwart der Ligand-Verbindung, hergestellt werden.

Der Wolframoxo(VI)-Komplex kann, wie in DE 25 16 698 beschrieben, vor der eigentlichen Reaktion hergestellt werden, oder aber auch gleich im Reaktionsgemisch hergestellt werden.

Beispielsweise kann man Natriumwolframatdihydrat als Feststoff zum Edukt-Allylalkohol, in diesem Fall Geraniol, zu geben. Durch Erwärmen der Mischung auf die für die Isomerisierung gewünschte Temperatur bildet sich der Isomerisierungskatalysator im Reaktionsgemisch. Das bei der Katalysator-Bildung frei werdende Wasser wird durch Reaktivdestillation mit dem Isomerisierungsprodukt, in diesem Fall Linalool, gemeinsam entfernt.

Der Gegenstand der DE 25 16 698 wird daher durch Zitieren in diese Patentanmeldung aufgenommen.

Im allgemeinen setzt man die Wolframoxo(VI)-Komplexe im Edukt-Allylalkohol gelöst in einer Konzentration von 0,001 bis etwa 5 Gew.-% ein.

Auch die Aminoalkohole werden im allgemeinen vorteilhaft im Edukt-Allylalkohol gelöst eingesetzt. Man verwendet sie im allgemeinen in einer Menge von von 1 Mol-% bis 1000 Mol-%, vorzugsweise von 33 Mol-% bis 500 Mol-%, bezogen auf die Wolframverbindung.

Die eingestellte Menge des Aminoalkohol-Liganden und insbesondere die Menge des Liganden im Verhältnis zur Menge des eingesetzten Wolframoxo(VI)-Komplexes hat Einfluß auf die Geschwindigkeit und die Selektivität der Reaktion.

Eine niedrige Aminoalkohol-Liganden-Menge bezogen auf die Wolfram-Menge, bewirkt eine hohe Reaktionsgeschwindigkeit, aber auch eine etwas niedrigere Selektivität, während eine hohe Liganden-Menge das Produkt mit hoher Selektivität, aber u.U. niedrigerer Raumzeitausbeute ergibt.

Die Aminoalkohole werden bevorzugt eingesetzt in einer Menge von 100 Mol-% bis 400 Mol-%, bezogen auf die Gesamt-Zahl der Ausgangs-Hydroxylgruppen im Aminoalkohol und auf die Wolfram-Menge, insbesondere in einer Menge von 300 Mol-% bis 400 Mol-%, bezogen auf die Gesamt-Zahl der Ausgangs-Hydroxylgruppen im Aminoalkohol und auf die Wolfram-Menge.

Die Absolutkonzentrationen von Ligand und Wolfram-Komplex in der Reaktionsmischung sind im erfindungsgemäßen Verfahren unkritisch und können beispielsweise derart erhöht werden, daß die Geschwindigkeit der Gleichgewichtseinstellung in gewünschter Weise gesteigert wird.

Eine Steigerung der Geschwindigkeit der Gleichgewichtseinstellung kann in dem erfindungsgemäßen Verfahren auch dadurch erzielt werden, daß gebildetes Reaktionswasser aus der Mischung entfernt wird, beispielsweise durch Überleiten eines Inertgasstromes, Zusatz bekannter Wasser entziehender Mittel oder Strippen mit dem Gasstrom während der Destillation.

In den Molframoxo (VI)-Komplexen können alle oder nur ein Teil der als Liganden fungierenden Alkohol-Reste am Wolfram-Atom und alle oder nur ein Teil der als zusätzliche Liganden fungierenden Stickstoff-Basen durch die als zusätzliche Liganden zugesetzten Aminoalkohole ausgetauscht werden.

Das erfindungsgemäße Verfahren wird im allgemeinen bei Temperaturen von 50 bis 300°C, vorzugsweise bei 150 bis 250°C, durchgeführt.

Es kann mit und ohne Mitverwendung eines Lösungsmittels, diskontinuierlich, aber auch kontinuierlich durchgeführt werden.

Als Lösungsmittel haben sich beispielsweise die hochsiedenden Nebenverbindungen der Allylalkohol-Isomerisierung als besonders vorteilhaft erwiesen.

Das erfindungsgemäße Verfahren kann vorteilhaft durchgeführt werden, wenn man die Edukt-Allylalkohole im Reaktionsgemisch in einer Konzentration von etwa 10 Gew.-% bis zu 100 Gew.-% vorliegen hat.

Die Aufarbeitung des Reaktionsgemisches erfolgt in an sich bekannter Weise.

Besonders vorteilhaft gestaltet sich das erfindungsgemäße Verfahren, wenn man als Edukt-Allylalkohole Geraniol und Nerol verwendet werden und als Produkt-Allylalkohol 2-Linalool herstellt.

Zur Aufarbeitung wird hierbei 2-Linalool als niedriger siedende Komponente durch Destillation vom Produktgemisch abgetrennt. Im allgemeinen werden Edukt-Allylalkohole und Nebenverbindungen im Produkt-Allylalkohol enthalten sein. Eine Reindestillation des Produkt-Allylalkohols kann nach bekannten Verfahren erfolgen.

Die Isomerisierung stellt eine Gleichgewichtsreaktion dar und die Gleichgewichtslage hängt von den thermodynamischen Eigenschaften der Edukt- und Produkt-Allylalkohole sowie von den Reaktionsbedingungen ab. Eine diskontinuierliche oder kontinuierliche Entfernung von Linalool, dem im Gemisch am niedrigsten siedenden Allylalkohol aus dem Reaktionsansatz erlaubt auch bei ungünstiger Gleichgewichtseinstellung aufgrund der Verschiebung des Gleichgewichtes eine günstige Raum-Zeit-Ausbeute, wobei der Sumpf der Destillationskolonne als Reaktionsraum dient.

Die folgenden Beispiele und Vergleichsbeispiele sollen das erfindungsgemäße Verfahren näher beschreiben.

### Vergleichsbeispiele 1* bis 5*

Die Versuche zur Isomerisierung von Geraniol und Nerol zu 2-Linalool wurden in einem 100-ml-Dreihals-Glaskolben als Reaktionsraum mit Innenthermometer, Destillationsbrücke, Gaszuleitungsrohr und einem Magnetrührer durchgeführt. Der Reaktionskolben wurde für die Reaktion in einem Silikonölbad beheizt und es wurde ein kontinuierlicher Strom von 0,3 l/h Argon oder Stickstoff über die gerührte Reaktionslösung geleitet. Sobald der Edukt-Allylalkohol die erforderliche Temperatur erreicht hatte, wurde eine berechnete Menge der Katalysator-Lösung zugegeben. Die Versuche wurden nach 1 h durch Abkühlen beendet und die Produkt-Zusammensetzung mittels gaschromatischer Analyse (GC) ermittelt. Während der Reaktionen wurde kein Produkt abdestilliert.
Die Katalysatorlösung wurde hergestellt, indem 2 g (5,9 mmol) wOCl₄ in 30 ml Toluol suspendiert wurden. Nach der Zugabe von 10 g (63 mmol) Tetrahydrogeraniol und dem Durchleiten eines schwachen Ammoniak-Stroms wurde über 10 g Kieselgel filtriert und das Kieselgel mit 5 ml Toluol nachgewaschen. Die vereinigten Filtrate ergaben die fertige Katalysatorlösung mit einem Gehalt von 0,13 M/l Wolfram.

Nach Beendigung der Reaktion durch Abkühlen wurde die Produkt-Zusammensetzung mittels GC bestimmt. Bei den in Tabelle 1 genannten Leichtsiedern handelt es sich um Dehydratisierungsprodukte aus Geraniol, Nerol und Linalool. Bei den in Tabelle 1 genannten Schwersiedern handelt es sich um Verbindungen, die durch Etherbildung aus Geraniol, Nerol und Linalool entstanden sind. Die Selektivität für Linalool wurde ermittelt aus dem Quotienten des Umsatzes zu Linalool und dem Gesamt-Umsatz von Geraniol. Das eingesetzte Geraniol hatte eine Zusammensetzung von 96 % Geraniol und etwa 4 % Nerol.

**Tabelle 1**

| Vgl.-Beispiele | Stickstoff enthaltende Base | Geraniol [GC-FI.-%] | 2-Linalool [GC-FI.-%] | Leichtsieder [GC-FI.-%] | Schwenieder [GC-FI.-%] |
|---|---|---|---|---|---|
| 1* | - | 50,7 | 17,7 | 5,4 | 18,1 |
| 2* | Diethylamin | 70,6 | 19.5 | 1,2 | 3,1 |
| 3* | Pyridin | 56,5 | 20,2 | 4,9 | 10,6 |
| 4* | Imidazol | 62,3 | 19,1 | 3,5 | 8,9 |
| 5* | Poly-(4-vinyl-pyridin) | 72,8 | 17,1 | 1,4 | 3,6 |

### Beispiel 1 und Vergleichsbeispiel 6*( analog DE 25 16 698)

### Einfluß eines Aminoalkohols als Liganden

Eine Mischung aus jeweils 20 g (0,13 mol) Geraniol, 1,0 ml (0,13 mmol) der vorbereiteten Wolframoxo(VI)-(tetrakisgeranylat)-Lösung sowie die aus der folgenden Tabelle 2 ersichtliche Stickstoff-Base Diethylamin und der Aminoalkohol 1-Amino-2 propanol in dem aus der Tabelle 2 ersichtlichen Molverhältnis wurden in einem 100-ml-Dreihals-Glaskolben für 1 h auf 200°C erhitzt. Eine anschließende GC der Reaktionsmischung ergab das aus der Tabelle 2 ersichtliche Ergebnis.

**Tabelle 2**

| Beispiele und Vgl.-Beispiele* | Mol-Verhältnisse Diethylamin: Wolfram: 1-Amino-2-propanol | Geraniol [GC-FI.-%] | Linalool [GC-FI.-%] | Leichtsieder [GC-FI.-%] | Schwersieder [GC-FI.-%] | Selektivität zu 2-Linalool [%] |
|---|---|---|---|---|---|---|
| 6* | 2:1:0 | 66,0 | 26,1 | 0,9 | 3,4 | 87 |
| 1 | 2:1-3,5 | 47,7 | 42,2 | 1,2 | 3,0 | 87 |

Die Beispiele zeigen, daß die Reaktion in Gegenwart von 1-Amino-2-propanol bei gleich guter Selektivität in Bezug auf das gebildete 2-Linalool deutlich schneller verläuft.

### Beispiele 2 a bis 2c

### Einfluß der Konzentration des Aminoalkohol-Liganden

Eine Mischung aus jeweils 20 g (0,13 mol) Geraniol, 1,0 ml (0,13 mmol) der vorbereiteten Wolframoxo(VI)-(tetrakisgeranylat)-Lösung sowie dem Aminoalkohol 1-Amino-2-propanol in dem aus der folgenden Tabelle 3 ersichtlichen Molverhältnis wurden in einem 100 ml-Dreihals-Glaskolben für 1 h auf 200°C erhitzt. Eine anschließende GC der Reaktionsmischung ergab das aus der Tabelle 3 ersichtliche Ergebnis.

**Tabelle 3**

| Beispiele | Mot-Verhältnis 2-Aminoethanol: Wolfram | Geraniol [GC-FI.-%] | Linalool [GC-FI.-%] | Leichtsieder [GC-FI.-%] | Schwersieder [GC-FI.%] | Selektivität zu 2-Linalool [%] |
|---|---|---|---|---|---|---|
| 2a | 3:1 | 71,5 | 20,1 | 0,6 | 1,5 | 82 |
| 2b | 4:1 | 75,4 | 17,4 | 0,5 | 1,3 | 84 |
| 2c | 5:1 | 76,8 | 15,8 | 0,4 | 1,0 | 82 |

Die Beispiele zeigen, daß die Reaktionsgeschwindigkeit bei zunehmender Menge von 2-Amino-ethanol in Bezug auf die Wolfram-Menge bei gleichbleibender Selektivität ab nimmt.

### Beispiele 3a und 3b

### Einfluß der Temperatur

Eine Mischung aus jeweils 90 g (0,59 mol) Geraniol, 1,5 ml (0,2 mmol) der vorbereiteten Wolframoxo(VI)-(tetrakisgeranylat)-Lösung sowie 1-Amino-2-propanol, der Stickstoffbase Diethylamin in dem Molverhältnis Diethylamin : Wolfram : 1-Amino-2-propanol = 2 : 1 : 3,5 wurden in einem 250 ml-Dreihals-Glaskolben für 1 h auf die aus Tabelle 4 ersichtliche Temperatur erhitzt.

Eine anschließende GC der Reaktionsmischung ergab das aus der Tabelle 4 ersichtliche Ergebnis.

**Tabelle 4**

| Beispiele | Temperatur (°C) | Geraniol (GC-FI.-%] | Linalool (GC-FI.-%] | Leichtsieder (GC-FI.-%] | Schwersieder (GC-FI.-%] | Selektivität zu 2-Linalool [%] |
|---|---|---|---|---|---|---|
| 3a | 180 | 82,5 | 7,6 | 0,3 | 1,2 | 56 |
| 3b | 190 | 69,4 | 15,7 | 0,7 | 1,5 | 59 |

Die Beispiele zeigen, daß bei Temperaturen tiefer als 200°C bei Verwendung von 1-Amino-2-propanol sowohl die Reaktivität als auch die Selektivität des Katalysators abnehmen.

### Beispiele 4a und 4b

### Einfluß eines Gasstroms auf die Reaktionsgeschwindigkeit

Eine Mischung aus jeweils 20 g (0,13 mol) Geraniol, 1,0 ml (0,13 mmol) der vorbereiteten Wolframoxo(VI)-(tetrakisgeranylat)-Lösung sowie die Stickstoff-Base Diethylamin und der Aminoalkohol Triethanolamin in dem Molverhältnis Diethylamin : Wolfram : Triethanolamin = 2 : 1 : 1 wurden in einem 250 ml-Dreihals-Glaskolben unter Überleiten eines kontinuierlichen Argonstroms für 1 h auf 200°C erhitzt. Eine anschließende GC der Reaktionsmischung ergab das aus der Tabelle 5 ersichtliche Ergebnis.

**Tabelle 5**

| Beispiele | Gasstrom | Geraniol [GC-FI.-%] | Linalool (GC-FI.-%] | Leichtsieder [GC-FI-%] | Schwersieder [GC-FI.-%] | Selektivität zu 2-Linalool [%] |
|---|---|---|---|---|---|---|
| 4a | ― | 76,4 | 16,3 | 0,3 | 1,0 | 83 |
| 4b | Argon | 40,9 | 45,6 | 1,6 | 5,0 | 83 |

Die Beispiele zeigen, daß im Reaktionsgemisch vorhandenes Reaktionswasser sich inhibierend auf die Isomerisierung auswirkt. Durch das Entfernen von Wasser mit Hilfe von einem Gasstrom wird die Isomerisierung beschleunigt.

## Patentansprüche

1. Verfahren zur Isomerisierung von Edukt-Allylalkoholen zu Produkt-Allylalkoholen in Gegenwart von vorgefertigten oder in situ erzeugten Wolframoxo(VI)-Komplexen enthaltend zusätzliche Stickstoff enthaltende Liganden bei Temperaturen von 50 bis 300°C, **dadurch gekennzeichnet, daß** in dem Katalysator neben den als zusätzliche Stickstoff enthaltende Liganden bekannten Stickstoffbasen oder anstelle dieser Stickstoffbasen als zusätzliche Stickstoff enthaltende Liganden Aminoalkohole enthalten sind.

2. Verfahren gemäß Anspruch 1, **dadurch gekennzeichnet, daß** man als Aminoalkohole Triethanolamin, Diethanolamin, Monoethanolamin, Tripropanolamin, Dipropanolamin, 3-Amino-1-propanol, 1-Amino-2 -propanol, 2-Amino-1-propanol, Butyldiethanolamin, Methyldiisopropanolamin, N-(2-Hydroxy-benzyl)-amin oder N,N'-Bis-(2-hydroxy-benzyl)-1,2-diaminoethan verwendet.

3. Verfahren gemäß Anspruch 1, **dadurch gekennzeichnet, daß** man den Wolframoxo(VI)-Komplex im Edukt-Allylalkohol gelöst in einer Konzentration von 0,001 bis etwa 5 Gew.-% einsetzt.

4. Verfahren gemäß Anspruch 1, **dadurch gekennzeichnet, daß** man den Aminoalkohol im Edukt-Allylalkohol gelöst in einer Konzentration von 1 Mol-% bis 1000 Mol-%, bezogen auf die Wolframverbindung, einsetzt.

5. Verfahren gemäß Anspruch 1, **dadurch gekennzeichnet, daß** man den Aminoalkohol im Edukt-Allylalkohol gelöst in einer Konzentration von 33 Mol-% bis 500 Mol-%, bezogen auf die Wolframverbindung, einsetzt.

6. Verfahren gemäß Anspruch 1, **dadurch gekennzeichnet, daß** in den Wolframoxo(VI)-Komplexen alle oder nur ein Teil der Wolfram-Alkoholat-Liganden durch die als zusätzliche Liganden zugesetzten Aminoalkohole ausgetauscht werden.

7. Verfahren gemäß Anspruch 1, **dadurch gekennzeichnet, daß** primäre oder sekundäre Edukt-Allylalkohole zu tertiären Produkt-Allylalkoholen isomerisiert werden.

8. Verfahren gemäß Anspruch 1, **dadurch gekennzeichnet, daß** die Isomerisierung bei Temperaturen von 150 bis 250°C durchgeführt wird.

9. Verfahren gemäß Anspruche 1, **dadurch gekennzeichnet, daß** die Edukt-Allylalkohole im Reaktionsgemisch in einer Konzentration von 10 Gew.-% bis zu 100 Gew.-% vorliegen.

10. Verfahren gemäß Anspruch 1, **dadurch gekennzeichnet, daß** als Lösungsmittel die hochsiedenden Nebenverbindungen der Allylalkohol-Isomerisierung verwendet werden.

11. Verfahren gemäß Anspruch 1, **dadurch gekennzeichnet, daß** als Edukt-Allylalkohole Geraniol und Nerol verwendet werden und als Produkt-Allylalkohol 2-Linalool hergestellt wird.

## Claims

1. A process for the isomerization of precursor allyl alcohols to product allyl alcohols in the presence of preprepared or in-situ-generated tungsten oxo(vI) complexes comprising additional nitrogen-containing ligands at temperatures of from 50 to 300°C, wherein, in the catalyst, as well as the nitrogen bases known as additional nitrogen-containing ligands, or instead of these nitrogen bases, aminoalcohols are present as additional nitrogen-containing ligands.

2. A process as claimed in claim 1, wherein the aminoalcohols used are triethanolamine, diethanolamine, monoethanolamine, tripropanolamine, dipropanolamine, 3-amino-1-propanol, 1-amino-2-propanol, 2-amino-1-propanol, butyldiethanolamine, methyldiisopropanolamine, N-(2-hydroxybenzyl)amine or N,N'-bis(2-hydroxybenzyl)-1,2-diaminoethane.

3. A process as claimed in claim 1, wherein the tungsten oxo(VI) complex is used dissolved in the precursor allyl alcohol in a concentration of from 0.001 to about 5% by weight.

4. A process as claimed in claim 1, wherein the aminoalcohol is used dissolved in the precursor allyl alcohol in a concentration of from 1 mol% to 1000 mol%, based on the tungsten compound.

5. A process as claimed in claim 1, wherein the aminoalcohol is used dissolved in the precursor allyl alcohol in a concentration of from 33 mol% to 500 mol%, based on the tungsten compound.

6. A process as claimed in claim 1, wherein, in the tungsten oxo(VI) complexes, all or only some of the tungsten alkoxide ligands are replaced by the aminoalcohols added as additional ligands.

7. A process as claimed in claim 1, wherein primary or secondary precursor allyl alcohols are isomerized to tertiary product allyl alcohols.

8. A process as claimed in claim 1, wherein the isomerization is carried out at temperatures of from 150 to 250°C.

9. A process as claimed in claim 1, wherein the precursor allyl alcohols in the reaction mixture are present in a concentration of from 10% by weight to 100% by weight.

10. A process as claimed in claim 1, wherein the solvents used are the high-boiling secondary compounds of the allyl alcohol isomerization.

11. A process as claimed in claim 1, wherein the precursor allyl alcohols used are geraniol and nerol, and the product allyl alcohol prepared is 2-linalool.

## Revendications

1. Procédé pour l'isomérisation d'alcools allyliques éduits en alcools allyliques produits en présence de complexes de tungstène-oxo(VI) préélaborés ou préparés in situ, contenant des ligands comportant de l'azote additionnel, à des températures de 50 à 300°C, **caractérisé par le fait que** sont contenus dans le catalyseur, outre les ligands contenant de l'azote additionnels, des bases azotées connues ou des aminoalcools à titre de ligands contenant de l'azote additionnels à la place de ces bases azotées.

2. Procédé selon la revendication 1, **caractérisé par le fait qu'**on utilise comme aminoalcools la triéthanolamine, la monoéthanolamine, la tripropanolamine, la dipropanolamine, le 3-amino-1-propanol, le 1-amino-2-propanol, le 2-amino-1-propanol, la butyldiéthanolamine, la méthyldiisopropanolamine, la N-(2-hydroxy-benzyl)-amine ou le N,N'-bis-(2-hydroxy-benzyl)-1,2-diaminoéthane.

3. Procédé selon la revendication 1, **caractérisé par le fait qu'**on utilise le complexe de tungstène-oxo(VI) dans l'alcool allylique éduit dissous en une concentration de 1 % en moles à 1000 % en moles, par rapport au composé de tungstène.

4. Procédé selon la revendication 1, **caractérisé par le fait qu'**on dissout l'aminoalcool dans l'alcool allylique éduit, à une concentration de 1 % en moles à 1000 % en moles, par rapport au composé de tungstène.

5. Procédé selon la revendication 1, **caractérisé par le fait qu'**on dissout l'aminoalcool dans l'alcool allylique éduit, à une concentration de 33 % en moles à 100 % en moles, par rapport au composé de tungstène.

6. Procédé selon la revendication 1, **caractérisé par le fait que** dans les complexes de tungstène-oxo(VI) la totalité ou seulement une partie des ligands d'alcoolate de tungstène est remplacée par les aminoalcools ajoutés à titre de ligands additionnels.

7. Procédé selon la revendication 1, **caractérisé par le fait que** des alcools allyliques éduits primaires ou secondaires sont isomérisés en alcools allyliques produits tertiaires.

8. Procédé selon la revendication 1, **caractérisé par le fait que** l'isomérisation est effectuée à des températures de 150 à 250°C.

9. Procédé selon la revendication 1, **caractérisé par le fait que** les alcools allyliques éduits sont présents dans le mélange à une concentration de 10 % en poids à 100 % en poids.

10. Procédé selon la revendication 1, **caractérisé par le fait qu'**on utilise comme solvant les composés secondaires à point d'ébullition élevé de l'isomérisation de l'alcool allylique.

11. Procédé selon la revendication 1, **caractérisé par le fait qu'**on utilise du géraniol et du nérol comme alcools allyliques éduits, et on prépare du 2-linalol comme alcool allylique produit.
